# EUROPEAN PATENT SPECIFICATION

(11) **EP 1 664 765 B1**
(45) Date of publication and mention of the grant of the patent: **29.08.2007**
(21) Application number: 03817382.9
(22) Date of filing: 14.07.2003
(51) Int. Cl.: G01N 33/487

(54) **LABEL-FREE METHOD FOR CLASSIFICATION AND CHARACTERIZATION OF CELLULAR EVENTS**
MARKIERUNGSFREIES VERFAHREN ZUR KLASSIFIZIERUNG UND CHARAKTERISIERUNG ZELLULÄRER EREIGNISSE
METHODE NE FAISANT PAS APPEL A DES ETIQUETTES POUR LA CLASSIFICATION ET POUR LA CARACTERISATION D'EVENEMENTS CELLULAIRES

(43) Date of publication of application: 07.06.2006
(73) Proprietor: MDS Inc., doing business as MDS Sciex, Concord, Ontario L4K 4V8 (CA)
(72) Inventor: LIU, Vivian, Burlingame,CA 94010 (US); FULLER, Chris, Oakland, CA 94619 (US); PITCHFORD, Simon, San Francisco, CA 94114 (US); VERDONK, Ed, San Jose, CA 95125 (US)
(74) Representative: Roberts, Mark Peter
(86) International application number: PCT/CA2003/001060
(87) International publication number: WO 2005/005979

(56) References cited:
- WO-A-03/016887
- US-A- 5 792 668
- US-B1- 6 377 057
- BAUMANN W H ET AL: "Microelectronic sensor system for microphysiological application on living cells" SENS ACTUATORS, B CHEM;SENSORS AND ACTUATORS, B: CHEMICAL 1999 ELSEVIER SEQUOIA SA, LAUSANNE, SWITZERLAND, vol. 55, no. 1, 1999, pages 77-89, XP004175066
- ASAMI KOJI ET AL: "Dielectric analysis of yeast cell growth" BBA - GENERAL SUBJECTS, ELSEVIER SCIENCE PUBLISHERS, NL, vol. 1245, no. 1, 1995, pages 99-105, XP002246663 ISSN: 0304-4165
- EHRET R ET AL: "Monitoring of cellular behaviour by impedance measurements on interdigitated electrode structures" BIOSENS BIOELECTRONICS;BIOSENSORS & BIOELECTRONICS 1997 ELSEVIER SCIENCE LTD, OXFORD, ENGL, vol. 12, no. 1, 1997, pages 29-41, XP002272321
- WOLF BERNHARD ET AL: "Biofunctional hybrid structures-cell-silicon hybrids for applications in biomedicine and bioinformatics" BIOELECTROCHEM BIOENERG;BIOELECTROCHEMISTRY AND BIOENERGETICS OCT 1998 ELSEVIER SCIENCE S.A., LAUSANNE, SWITZERLAND, vol. 46, no. 2, October 1998 (1998-10), pages 215-225, XP002265839
- "Signal Transduction Assays" APPLIED BIOPHYSICS, [Online] 15 July 2002 (2002-07-15), pages 1-2, XP002272322 New York Retrieved from the Internet: <URL:http://www.biophysics.com/> [retrieved on 2004-03-02]

## Description

The present invention is related to methods for label-free interrogation and characterization of the physiological responses of cells using electromagnetic energy.

In particular, the present invention allows monitoring of specific receptor activation from all classes of receptors following perturbation of the cell in real-time without the use of tracer molecules or the need for system enhancements (such as transfection and/or overexpression) by monitoring cellular physiology (through electrical properties) in a single assay format. Although the method may be used for many classes of cell surface and cytoplasmic receptors, we have focused our discussion only on two classes of cell surface receptors: G-protein coupled receptors (GPCRs), and Protein Tyrosine Kinase receptors (PTKRs). Within the field of GPCRs, three main families have been described. These families are classified according to the G-protein that mediates the primary signal transduction pathway used by the GPCR. They are designated Gi, Gs, and Gq. Subtypes of each of these families exist, however, for studies demonstrating the usefulness of this invention, we have used ligands that stimulate only one subtype of receptors of the Gi, Gs, Gq, or PTKR classes. The primary signaling pathways activated by stimulation of these three receptor families are, 1) for Gi-coupled receptors, a decline in intracellular 3',5' cyclic adenosine monophosphate (cAMP), 2) for Gs-coupled receptors, an increase in cAMP and, 3) for Gq-coupled receptors, a increase in intracellular calcium ions.

Experiments linking bioimpedance and cellular analysis have been described in the scientific literature. Existing cellular bioimpedance measuring devices that monitor receptor stimulation do so using only morphological changes or usually only one frequency. Gheorghiu revealed that α- and β-dispersions, which appear from 100Hz to 10KHz and from 100kHz to 10MHz, respectively, should both be considered in any model of the dielectric behaviour of a cell (Gheorghiu; "Characterizing Cellular Systems by Means of Dielectric Spectroscopy" (Bioelectromagnetics 17:475-482 (1996)). α-dispersion information enables the evaluation of the biological cell resting potential and cell morphology, while information on the permittivity and the conductivity of cellular subcompartments - for example the cell membrane, the cytoplasm- are revealed only in the β-dispersions range. Given the known biochemical changes in both the cell membrane and the cytoplasm upon binding of a ligand to a membrane receptor, a system capable of making bioimpedance measurements across both the full α- and β-dispersions frequency range is of utility in the field of drug discovery (Smith, Duffy, Shen, and Oluff; "Dielectric Relaxation Spectroscopy and Some Applications in the Pharmaceutical Sciences" (Journal of Pharmaceutical Sciences 84:1029-1044 (1995)); Foster and Schwann; "Dielectric Properties of Tissues and Biological Materials: A Critical Review" (Critical Review in Biomedical Engineering 17:25-104 (1989))).

Wegener et al. discuss experimentation which involves the use of electrical impedance measurements to monitor β-adrenergic stimulation of bovine aortic endothelial cells (where β-adrenergic is a receptor for the Gs subtype) (Eur. J. Physiol (1999) 437:925-934), but present only single frequency impedance data after concluding that a multi-frequency scanning mode was not as well suited for monitoring fast changes of cellular properties as a single frequency mode and do not present a method of classification of receptor responses. Their chosen 10 KHz operating frequency seemingly weights their measured cell response to being sensitive primarily to changes in the α-dispersion parameters, not the β-dispersion parameters, a point that the authors acknowledge in discussing their results. Their results are explicitly attributed to modulation of the ion currents between the cells and the substrate they adhere to and to currents in the paracellular spaces.

Technologies coupling cellular activity and bioimpedance measurements would have applications in the area of drug discovery. Many technologies exist within this field for making cellular measurements. Most of the current technologies, however, utilize an optical or radio label as a key component of their detection scheme. One example of technology to monitor receptor-ligand binding is the FLIPR device offered by Molecular Devices. This device uses a fluorescent probe to detect the release of calcium inside a cell in response to stimulation of Gq linked GPCR's. The FLIPR technology uses this fluorescent probe to achieve the amplification necessary to detect a response. However, inherent background fluorescence of cells and cell culturing materials is a drawback along with the fact that only the Gq pathway is easily interrogated with this approach.

Another example of technologies applied to this field is the use of stably transfected reporter gene systems for the characterization of GPCR's as offered by Vertex Pharmaceuticals. These systems use a promiscuous G-protein that links the multiple GPCR subtypes (Gi, Gs, or Gq) through one pathway (typically linked through luciferase or beta-lactamase expression) so that any G protein can be characterized through the measurement of light output alone. These systems are often applied to ligand fishing for the orphan GPCR (these are GPCR's whose ligand is unknown) obviating the requirement for multiple assay platforms to detect the different signaling pathways. These systems suffer from two major drawbacks. First, light 5 output from the reporter gene product may be quenched within the cell, thus requiring high levels of expression of the receptor in order to achieve reasonable detection. Second, once the ligand for the orphan GPCR is discovered, determining the actual signal transduction pathway takes a considerable amount of additional work.

WO03016887 discloses a label-free method for the detection of a change in cellular activity as a result of the addition of a test substance to the medium in which the cells are located. According to this method microwaves are applied to the cells by means of an applicator comprising for example a microstrip or a coplanar waveguide. The response of this applicator to microwaves is measured for example by means of a network analyzer at a plurality of frequencies. WO03016887 discloses that the method could be used in a typical drug-discovery process (e.g. hit detection, lead discovery, or lead optimization). In one specific embodiment the muscarinic m1 receptor that had been transfected into CHOk1 cells (Chinese Hamster Ovary wild-type cells) to form CHOm1 (transfected) cells was activated in the presence of an agonist. In one such assay, CHOm1 and CHOk1 cells were treated with carbachol, a known activator of the m1 receptor. Measurements were made over the range from 50 MHZ to 1 GHz. In a first series of measurements, taken 7 minutes after addition of carbachol and plotted over the indicated frequency range, un-transfected cells (CHOk1) were similar to controls, while transfected (CHOm1) cells treated with 10 uM carbachol showed a significant change in signal at all frequencies over the tested range. In some cases the signal over the 50 MHZ to 1 GHz range was integrated and the resulting integral for the signal of a test compound/cellular system was compared to the integrated signal for a buffer/cellular system. In one such assay, CHOk1 cells treated with various concentrations of carbachol showed essentially no change in signal over time, while CHOm1 transfected cells showed a typical dose/response effect of increased activity with time after addition of various concentrations of carbachol. According to WO03016887 this method can thus be used to detect and distinguish agonist and antagonist activity.

Accordingly, a need exists to develop label-free, bioimpedance characterization methods which measure across a frequency range which is large enough to yield both α- and β-dispersion information so that the physiologic response of a cell to stimulation is more accurately characterized. The present invention relates the details of the changes in electrical properties to the family of the second messenger pathway triggered, and demonstrates the ability to classify pathways based on changes in cellular electrical properties in the absence of any prior knowledge of the receptor or pathway under evaluation. The ability to assign an unknown ligand to an interaction with a specific pathway represents a major benefit over existing technology. No one existing technology can classify all of these signal transduction pathways simultaneously. With our system, pathway classification for the orphan receptor is achieved simultaneously with the discovery of the ligand and without a need to create stably transfected cell lines containing reporter gene systems. In addition, unlike other characterization technologies, the present invention does not need to use labels to detect stimulation of pathways, and does not need to artificially amplify receptor number by transfection in order to detect a response.

The invention is defined in the claims.

The invention will be further described, by way of example only, with reference to the accompanying drawings, in which:-
Figure 1a. A block diagram that illustrates one embodiment of the bioimpedance measuring system.
Figure 1b. illustrates a bioimpedance system which is used in accordance with one embodiment of the characterization method.
Figure 2. illustrates the steps of the cellular bioimpedance characterization method.
Figure 3. displays raw impedance magnitude differences for four major receptor families.
Figure 4. displays Legendre parameter graphs for three Gs receptors on CHO cells.
Figure 5. displays Legendre parameter graphs for three Gq receptors on CHO cells.
Figure 6. displays a comparison of parameter graphs for Gs and Gq receptors on CHO cells.
Figure 7. displays Legendre parameter graphs for three Gq receptors on HeLa cells.
Figure 8. displays Legnedre parameter graphs for two Gi receptors on HeLa cells.
Figure 9. displays Legendre parameter graphs of three PTKR receptors on HeLa cells.
Figure 10. displays a comparison of parameter graphs for Gq and Gi receptors on HeLa cells.
Figure 11. displays a comparison of parameter graphs for Gq an PTKR receptors on HeLa cells.
Figure 12. displays a comparison of parameter graphs for Gi and PTKR receptors on HeLa cells.
Figure 13. displays an analysis matrix for data classification for CHO cells.
Figure 14. displays an analysis matrix for data for HeLa cells.

The present invention is a method for the classification of different cellular events, such as activation of a signaling pathway. In particular, it enables the assignment, in real time, of a specific second messenger pathway to an unknown ligand/receptor pair, without the use of molecular labels. The classification is based on changes in the electrical properties of the cell. Living cells are incorporated into an electrical circuit.

The properties of this circuit are influenced by these cells. These properties can be measured by multiple frequency measurements over a range of frequencies. It is worth noting that cellular events may change the cell-circuit interactions as well as the electrical properties of the cell. The changes in cell-circuit interactions could include changes in the details of the cell positioning and attachment with respect to the electrodes or changes in cell morphology. These cellular event induced changes in cell-circuit interactions may contribute to the classification of the cellular events. Frequencies of the electromagnetic spectrum (e.g., microwave, radio-wave, audio, IR, optical, x-ray) are used. To facilitate throughput, these electrical circuits can be incorporated into the wells of a microtitre plate. In one embodiment, a 96-well microtitre plate is used. Alternative embodiments incorporate plates of various numbers of wells. Data may be collected before, during, and after the addition of a specific receptor ligand or other stimuli. If the cells respond to this stimulus, the measured electrical properties will also change.

In one embodiment, our electrical circuit consists of interdigitated coplanar electrodes patterned on the bottom of a 96-well microtitre plate, although additional 2-D and 3-D embodiments may be used, such as co-planar waveguides, coaxial electrodes, parallel plate electrodes, or any other microscopic or macroscopic electrode geometry commonly used to probe electrical properties of a solid or liquid sample. Although alternative electrode types may be used, in one embodiment we have used interdigitated coplanar electrodes because they have a greater region of sensitivity when compared to other electrode types and are less sensitive to changes in well diameter.

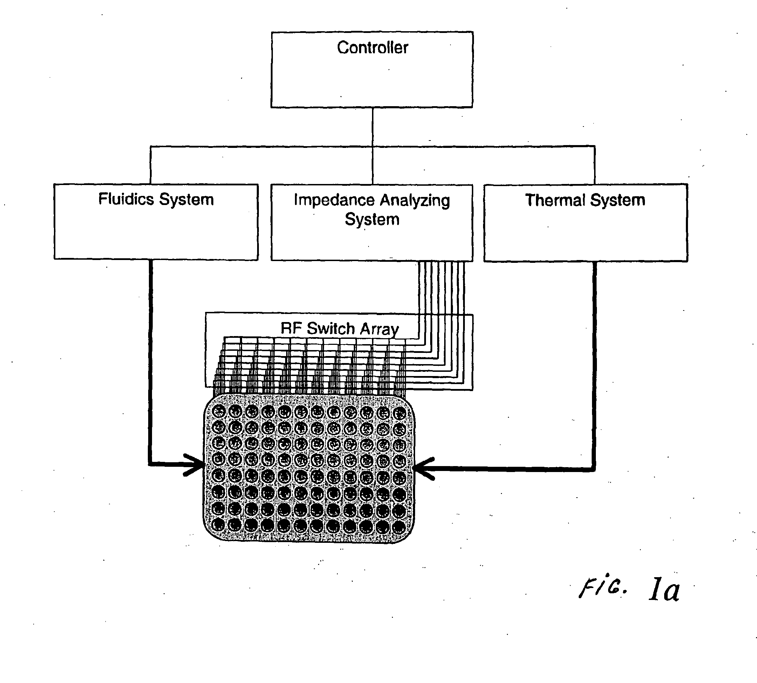

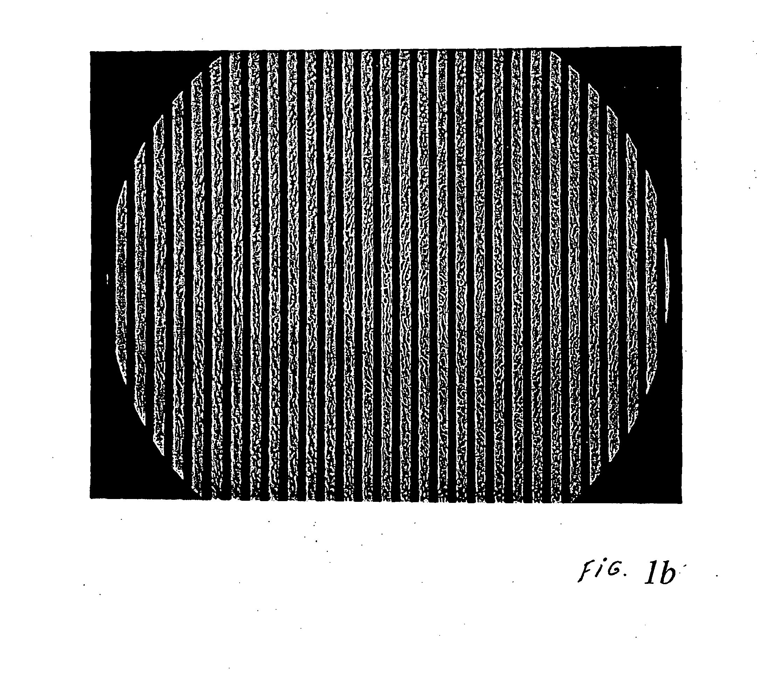

FIGURE 1. Cells, in a physiological buffer, are plated onto the surface partially covered by the electrodes and each well can be connected to one or more impedance analyzers through a signal multiplexer. Using the impedance analyzer, both the impedance magnitude and phase, over a frequency range of 40 Hz to 110 MHz, are recorded at periodic intervals. These frequencies were the full range capable on the Agilent impedance analyzer used, and spans the typical alpha and beta dispersion range for cells in physiological buffer. Additional information (dispersions) can be obtained at higher frequencies, including the gamma dispersions from bound water (GHz ranges), but become more difficult with the existing device architecture due to microwave resonance effects. Alternative embodiments utilize different frequency ranges.
Representative frequency ranges from alternative embodiments include 10Hz to 1000GHz, 100Hz to 500GHz, and 100Hz to 1GHz.

Upon interaction of the ligand with its receptor, the cell undergoes physiological changes over time that alter the measured electrical properties. As stated above, changes in the cellular electrical properties are sufficient for distinguishing which second messenger pathway has triggered the cellular changes. In one embodiment of our method, the needed cellular electrical property information is present in the changing impedance. In alternative embodiments, similar data sets may be used; for example, if one recorded complex reflection coefficients (S parameters), one would have the same information, as S parameter data can be converted to impedance data. Other examples include the measurement of resistance, reactance, admittance, conductance, or susceptance.

In other alternative embodiments, the classification is performed using other information (impedance at fewer frequencies, impedance magnitude only, impedance phase only, or properties such as total circuit resistance or capacitance, or changes in circuit voltage or current). While thorough studies still need to be completed, preliminary evidence suggests that simpler measurements will be sufficient for classification of the pathway.

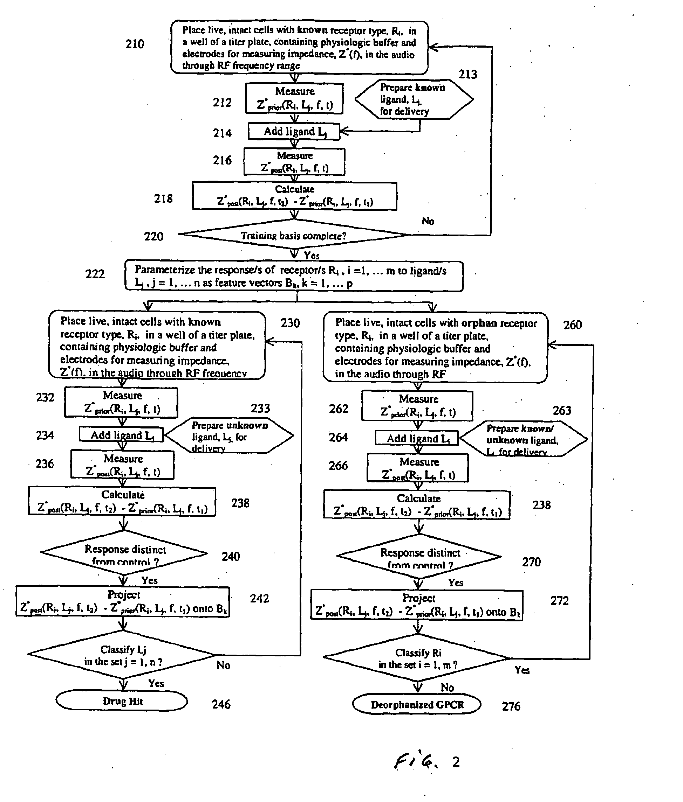

In one representative embodiment of the invention, the impedance data is processed in the manner shown in Figure 2. First, for each time point we measure the impedance over a range of frequencies after placing cells with known receptor types in a well of a micro titer plate (element 210 in Figure 2). Second, we select a time point corresponding close to but just preceding substance addition and measure the "reference" impedance 212. In the embodiment of Figure 2, the substance is a drug of interest, but in alternative embodiments, the substance may be a specific ligand, a protein, a lipid, a carbohydrate, a nucleic acid, water, an ion, or any other substance of interest. After drug addition (213, 214), at each subsequent time point for each frequency, we measure the impedance over a range of frequencies 216 and subtract the "reference" impedance magnitude and phase 218. Our data now consists of changes in impedance magnitude and phase for each frequency induced by the drug addition. Fourth, the changing impedance spectra are then fitted using the Legendre Polynomials to parameterize the cellular response 222. Specifically, in one embodiment, at each time point the change in impedance magnitude is fit to 7 Legendre polynomials and the change in impedance phase is fit to 7 Legendre polynomials. At each time point we then have 7 coefficients from the magnitude data and 7 coefficients for the phase data. If one plots these sets of coefficients as a function of time, the graphs contain "kinetic" trends that by eye can often be associated with specific second messenger pathways. The objective quantitative classification performed by a computer, however, does not rely on these patterns and trends. Rather, the computer algorithm needs only the coefficients at one time point after drug addition (corresponding to impedance data collected at this time and at the selected pre-drug addition time). The set of 7 magnitude coefficients and 7 phase coefficients are then compared to coefficient sets of known pathways (from a training data set) and assigned to a known pathway using standard multidimensional data classification algorithms. Alternative embodiments may contain coefficients fit to more or less Legendre polynomials, or time-dependent feature vectors parameterized with alternative methods. Using impedance data, the ligand-receptor interaction can be classified into one of the four categories, Gi, Gs, Gq, or PTKR. Thus, we have created a tool that can now subsequently assign an unknown ligand to an interaction with a receptor of the aforementioned classes.

### UNKNOWN LIGAND DISCOVERY

In one representative embodiment of the method to assign an unknown ligand of potential pharmaceutical activity, after placing cells with a known receptor type in a well of a microtitre plate (element 230 of Figure 2), the "reference" impedance magnitude and phase over a range of frequencies are measured at a time point corresponding to close but just preceding drug addition 232. After the drug containing the ligand is added 233, 234, the impedance magnitude and phase over a range of frequencies is measured at each subsequent time point for each frequency 236, and the "reference" impedance magnitude and phase are subtracted 238.
Next, the parameterized coefficients of the cellular response to the unknown ligand are calculated and compared to the coefficients for known ligands 240, 242, 244. The classification of the cellular response to the unknown ligand using a known receptor/ligand interaction yields valuable information regarding the cellular response, the stimulated receptor subtype, and the second messenger pathway.

### ORPHAN RECEPTOR DISCOVERY

Similarly, the exposure of intact cells with orphan or unknown receptors to potential ligand compounds allows for discovery of the ligand for the orphan thus de-orphanizing the receptor. Simultaneously the signal transduction pathway utilized by the receptor is ascertained by comparison to known patterns derived from training sets.(260-276).

### DISCUSSION OF EXPERIMENTAL DATA

The studies discussed here focused on the analysis of receptors of the follow types. In the category of GPCRs, Gi, Gs, and Gq related receptors were studied. In addition, several PTKR receptors were studied. Gi, Gs, and Gq receptors were studied in Chinese hamster ovary cells (CHO), while Gi, Gq, and PTKR receptors were studied in human cervical adenocarcinoma cells (HeLa). The data is presented in three categories. The first category is raw impedance difference data to show a sample of the actual data. The second category is Legendre polynomial parameterized data, and the third category is multidimensional classification data.

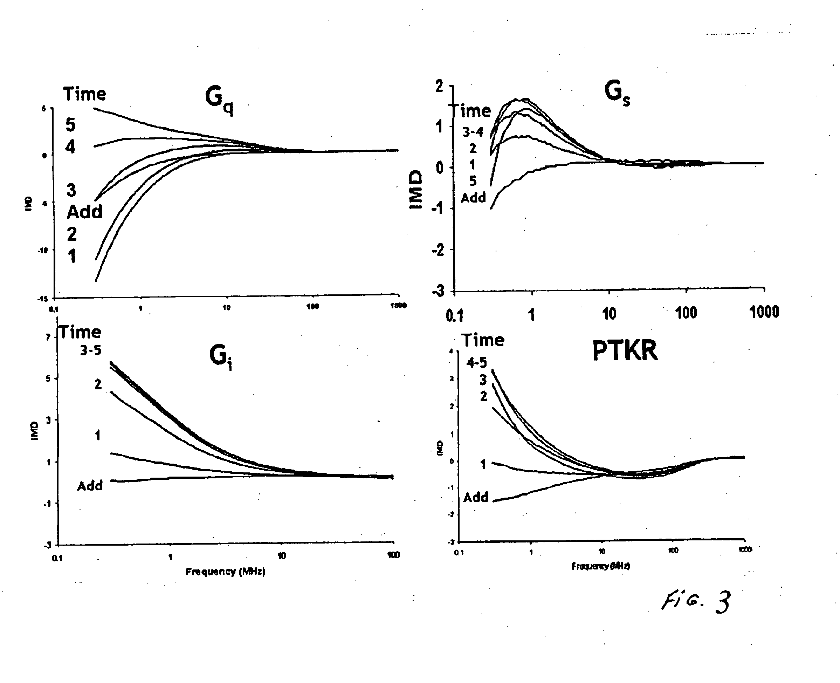

Cells are plated into the wells of 96-well microtitre plates containing interdigitated electrodes fused to the bottom of the wells. The cells are plated in standard tissue culture media containing serum and allowed to incubate and adhere overnight in a standard 37oC CO2 incubator. The next day the plates containing adhered cells are rinsed with HANKS balanced salt solution containing 10 mM HEPES (HH). HH is then added to the wells and the plates are allowed to equilibrate to room temperature for 1hr. The plates are then moved onto the reading instrument and baseline readings are taken for 15 min. At the end of the 15 min timepoint, ligands for the various receptors are added to the appropriate wells and readings are taken every 20 sec for 30 min. The pre-addition 15 min baseline readings are subtracted from the post-addition readings and the result is plotted as the raw impedance magnitude difference. Figure 3 shows the results obtained when ligands for Gq, Gi, Gs, GPCRs, and for a PTKR are added to cells. The numbers next to the traces refer to the number of minutes that have elapsed after ligand addition. As can be seen in this figure, each of the ligands produces characteristically different responses in the impedance magnitude difference.

This figure represents a sample of the actual data obtained. Quantitative analysis of this data proved difficult and therefore a means was developed to parameterize the data using the Legendre polynomials.

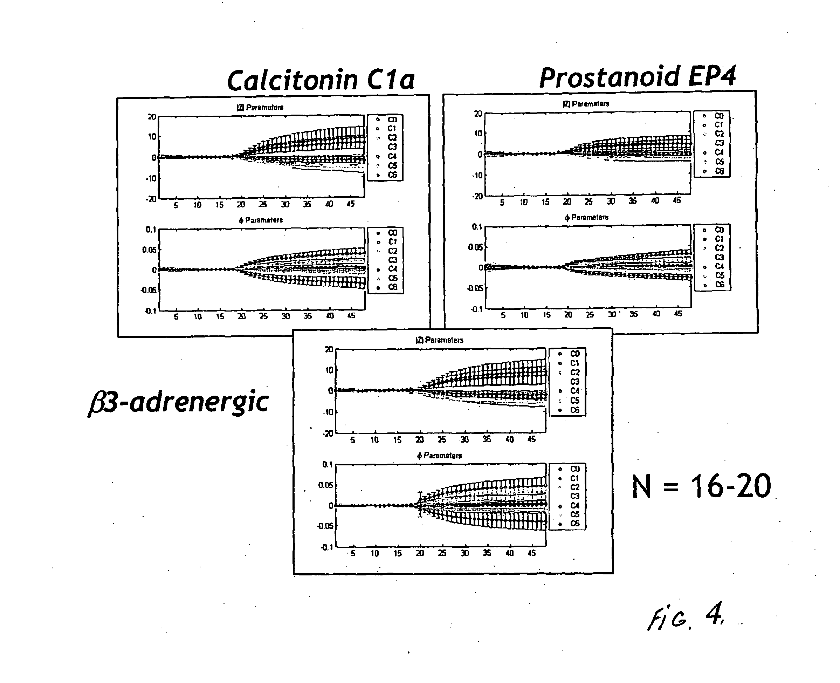

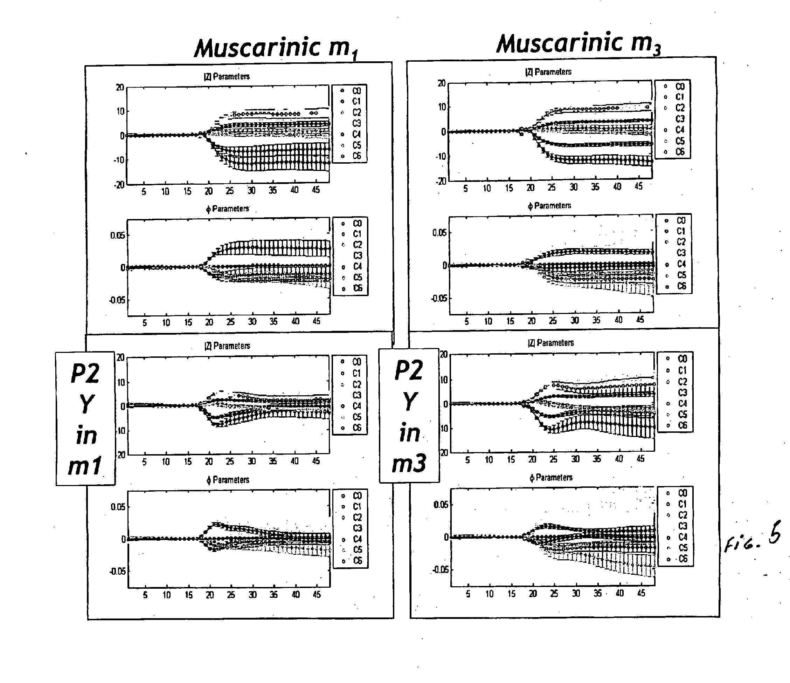

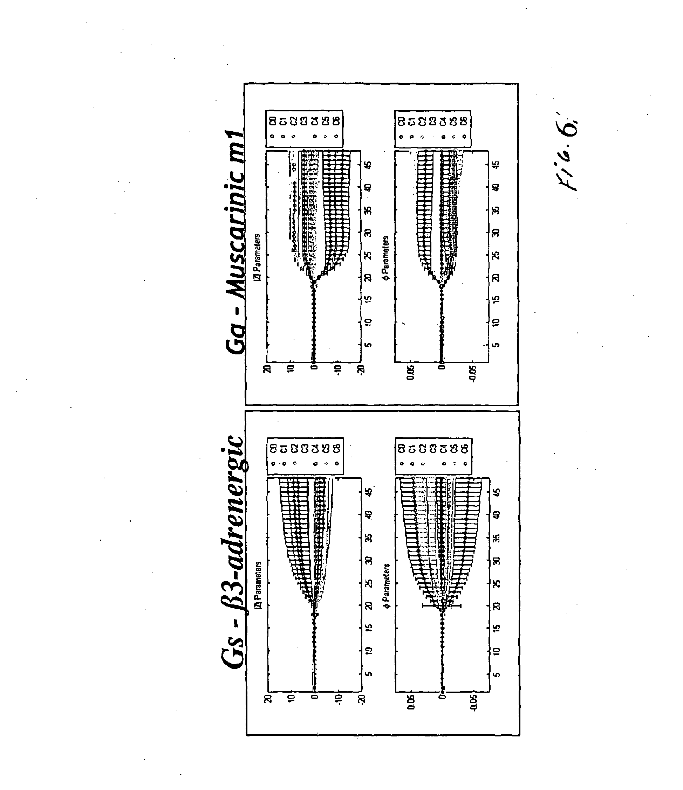

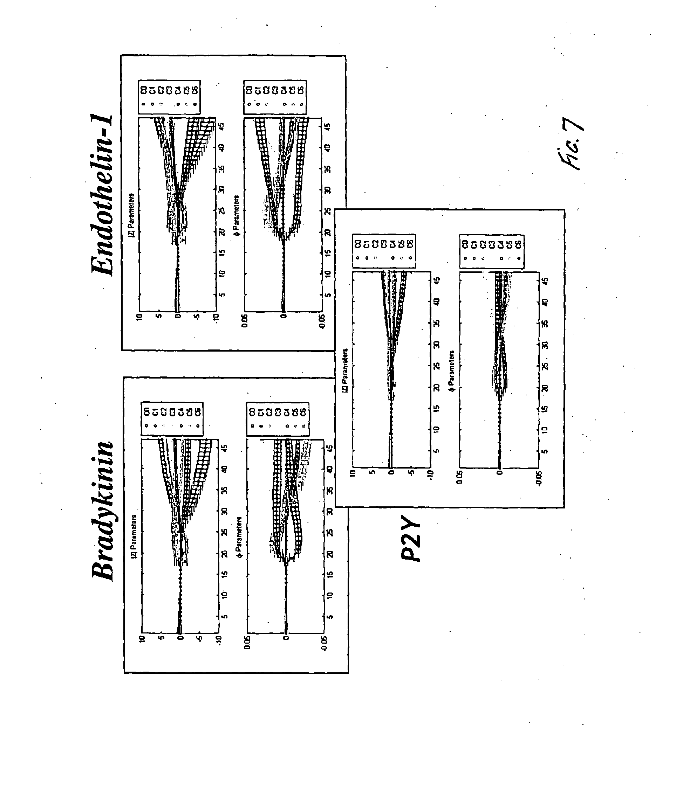

Legendre parameter graphs are presented in Figures 4-12. Figures 4, 5, and 6 represent data collected using CHO cells and Figures 7-12 represent data collected using HeLa cells. In Figure 4, three different ligands that stimulate three different Gs receptors were used to generate the data. The receptors were the calcitonin Cla (endogenous), the prostanoid EP4 (endogenous), and the beta3 adrenergic (transfected) receptor. As described previously, the transformation analysis produces 7 parameters for the impedance magnitude and 7 parameters for the impedance phase. These parameters are labeled C0-C6 on the graphs. For each receptor, a set of two graphs is seen. The top graph shows the Legendre parameters for the impedance magnitude and the lower graph shows that for the impedance phase. In this figure the magnitude in the change of the parameter value on the y-axis is plotted against time (from the start of the baseline readings) on the x-axis. The data is presented as the MEAN±S.D. There was an N of 16-20 for each data point. When the data is transformed in this way, clear patterns emerge. The patterns seen here in this figure were then designated as the Gs patterns for CHO cells. This figure is representative of the kinds of patterns that are produced using this analysis method. Figure 5 shows the results of a similar transformation but this time using ligands for the muscarinic M1 (transfected), muscarinic M3 (transfected), and P2Y (endogenous), Gq receptors in CHO cells. As can be seen, clearly distinct Gq patterns emerge from the graphs. The patterns seen in this figure were then designated as the Gq patterns for CHO cells. Figure 6 shows a side-by-side comparison of one set of Gs patterns with one set of Gq patterns. The differences between the patterns are significant and provide the first clear demonstration of the utility of our method.

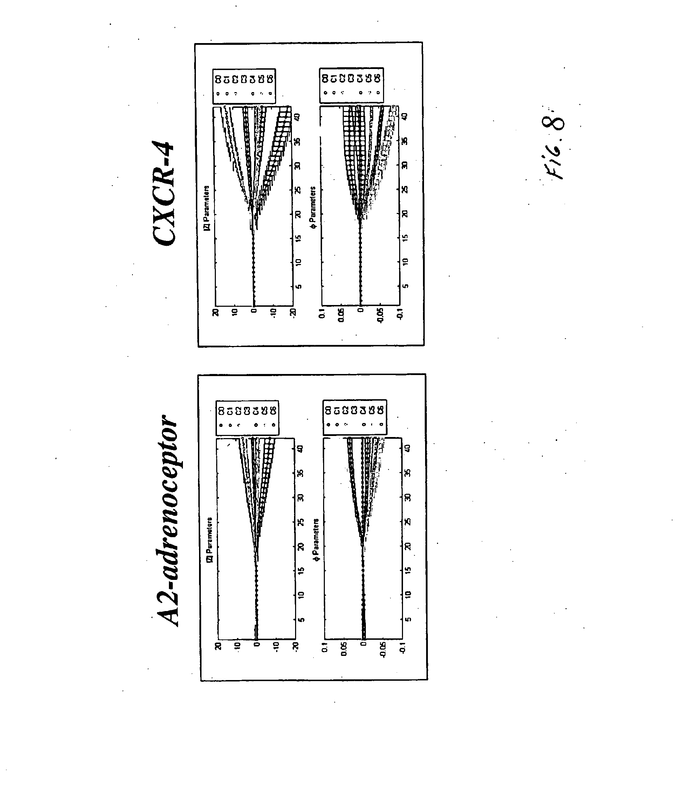

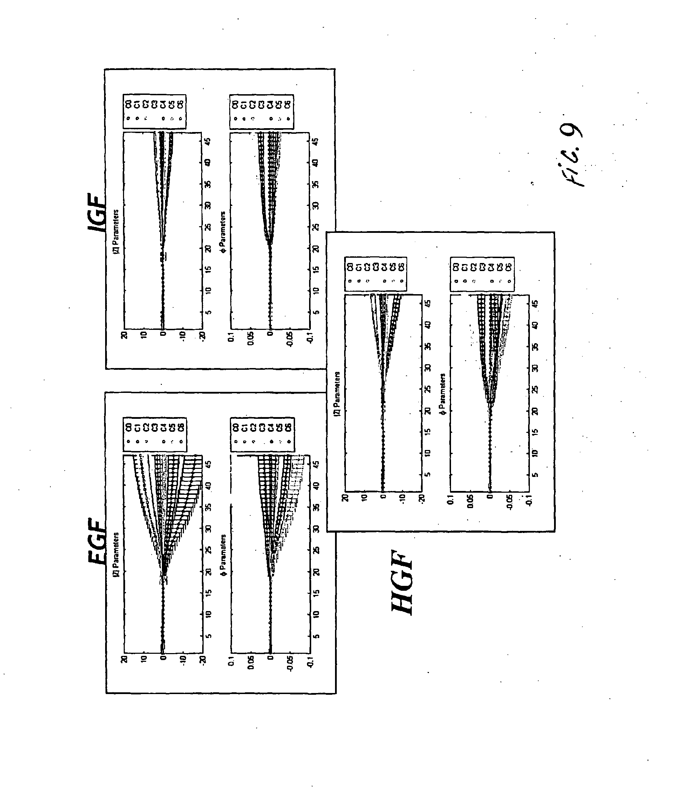

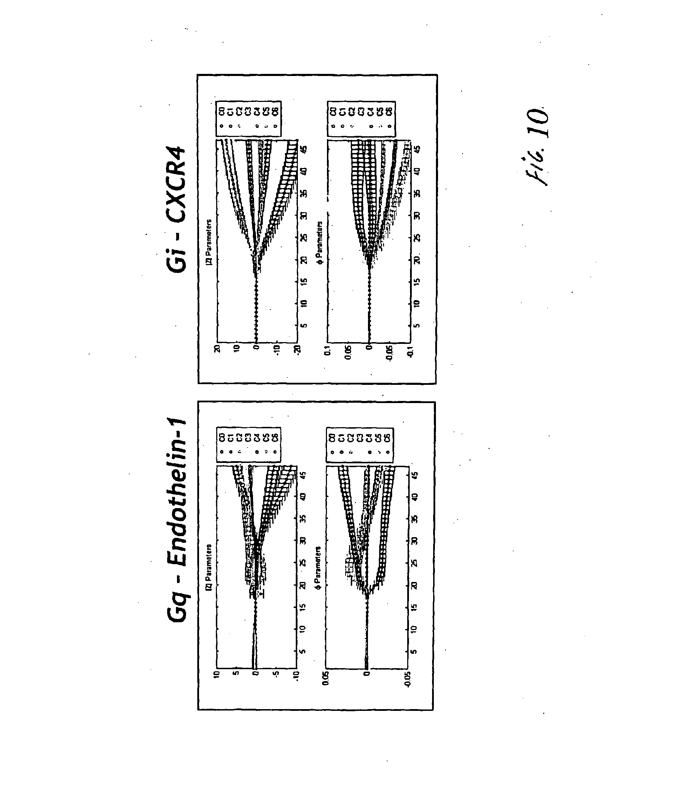

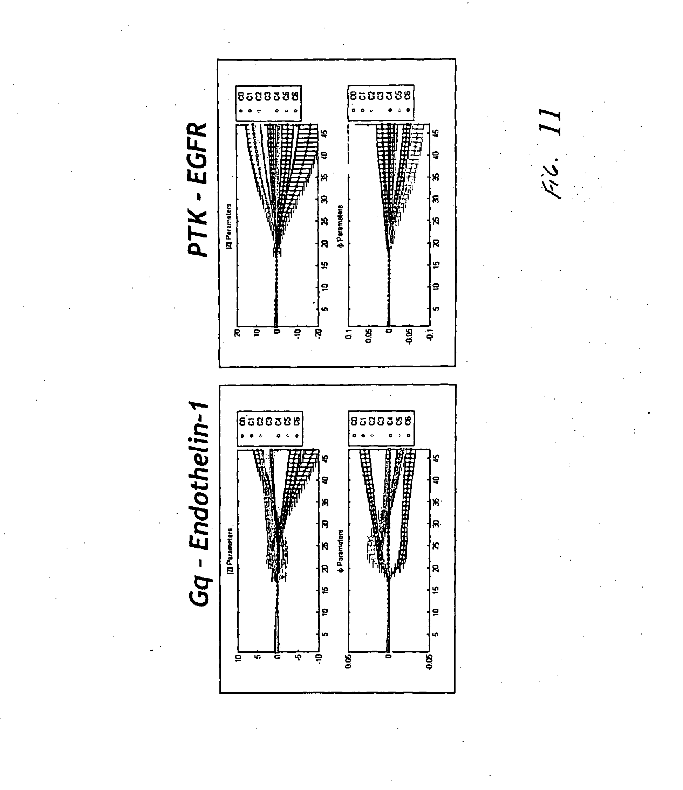

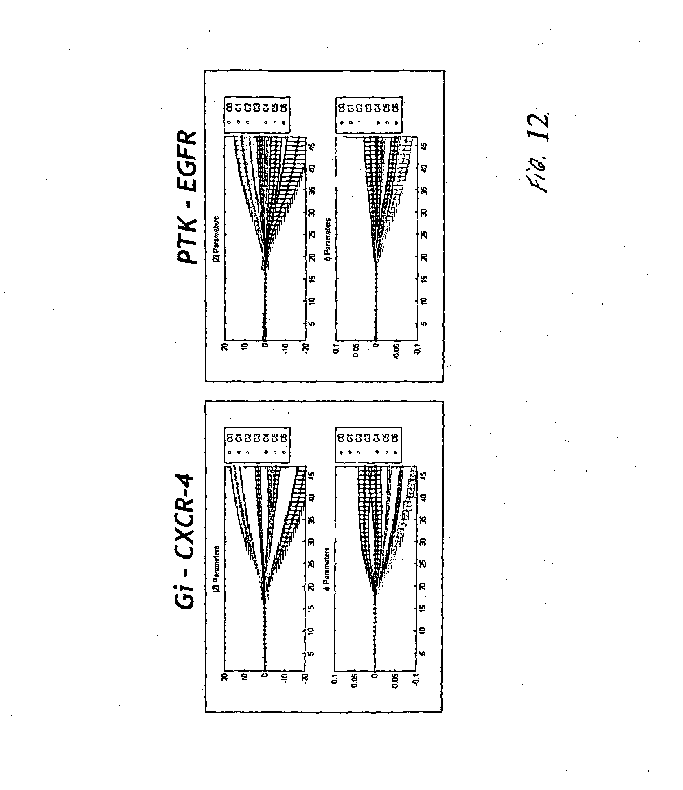

In Figures 7-9, similar transformations of data from HeLa cells stimulated with ligands for three Gq receptors (bradykinin, endothelin-1, and P2Y (all endogenous); Figure 7), two Gi receptors (alpha2-adrenoceptor, CXCR-4 (both endogenous); Figure 8), and three PTKR receptors (epidermal growth factor (EGF), insulin-like growth factor (IGF), and hepatocyte growth factor (HGF) (all endogenous); Figure 9) were performed to extend the results to an additional cell line and to include another receptor family, the PTKRs. The results show distinct patterns for each type of receptor within the cell line. This distinctiveness is evident in the side-by-side comparisons shown in Figures 10-12. Figure 10 shows the comparison of one set of patterns for Gq with one Gi set. Figure 11 shows the comparison of that same set of Gq patterns with that produced by a representative PTKR. And in Figure 12 the last comparison is shown between the Gi set and the PTKR set. These data.clearly show that the distinct intracellular pathway activated by these ligands are manifesting themselves as distinct patterns in graphs of the Legendre parameters. This correlation is the basis for our classification scheme. Stimulation of an unknown receptor, leading to production of a pattern similar to those presented, can easily be classified as using the intracellular pathway identified by that pattern.

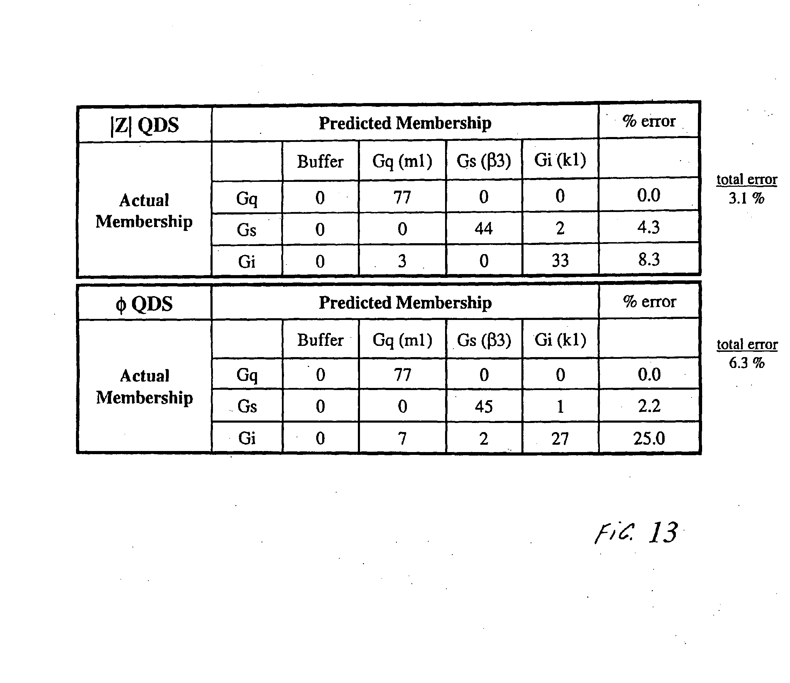

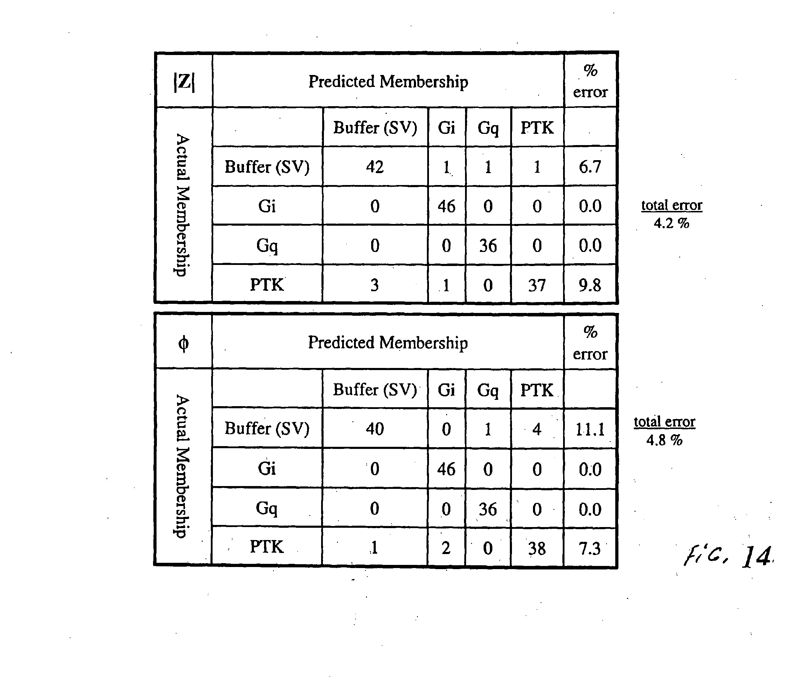

To verify what can be concluded by visual inspection of the parameter pattern results, the data was subjected to standard multidimensional data classification techniques. These analyses are presented in Figures 13 and 14. Training sets of pattern data were collected and fed into the process. Then, subsequent data was tested to see whether the classification technique could assign the proper second messenger pathway utilized (receptor type) to the data. Figure 13 shows the analysis of the CHO data and Figure 14 shows the analysis of the HeLa data. In both cases the algorithms were easily able to predict the receptor type with relatively low error rates. Thus we show that this cellular analysis method can easily and robustly predict the second messenger pathway utilized by a receptor from the pattern data.

### APPLICATIONS OF THE TECHNOLOGY

The here-in described label-free classification and characterization method can be used in a variety of applications:

### CASE #1: HIT IDENTIFICATION FOR AGONISTS OF KNOWN TRANSFECTED RECEPTORS

In this case the method is used for hit identification for agonists of known receptors. The question being asked by a pharmaceutical company is: Are there agonists for known receptors contained within the company's compound library?

The example we use here involves transfected G-protein coupled receptors (GPCRs) (other types of receptors can be utilized, depending on the need of the customer), and the procedure is as follows. The receptors of interest are transfected into a cell line known to have the second messenger apparatus necessary to transduce signals from the receptor. A parental cell line is transfected with a nonsense sequence so as to be used as a control for the transfection process during analysis of data. Unknown compounds (potential ligands) are then tested against both the sense and nonsense transfected cell lines. When stimulation is observed in the sense transfected group and not in the nonsense group, a hit is obtained. The reaction strength of the hit would be compared to the maximal reaction produced with known receptor agonist.

The advantages of our method in this use case are the following. Our method requires minimal assay development with no need for reporter systems to see the response of the transfected receptor, no need for additional reagents or fluorescent tags, and can be used with many different kinds of receptors including GPCRs, protein tyrosine kinase (PTKR), and nuclear receptors. No other single method or instrument for the analysis of GPCR second messenger pathways can interrogate all three (Gi, Gs, and Gq) pathways on the same platform, as this can. In addition, it can be used with adherent and nonadherent cells.

### CASE #2: HIT IDENTIFICATION AND PATHWAY CLASSIFICATION FOR AGONISTS OF UNKNOWN TRANSFECTED RECEPTORS

In this case again we will describe hit identification, but now we will add a description of the power of our method for pathway classification for agonists of unknown transfected receptors. For this case we will describe the de-orphanizing of orphan GPCRs.

The receptors of interest here are orphan GPCRs (oGPCRs). These are receptors that were discovered during sequencing of the human genome and that are potentially extremely important mediators of many disease processes. At present, their ligands are not known and the second messenger pathways they utilize are also unknown. In this case we establish again a database of patterns (a training set) for HEK293 cells (or any other cell line, depending on the need of the customer) and include patterns produced during stimulation with ligands specific for Gi, Gs, or Gq GPCRs. The oGPCR gene is transfected into HEK293 cells and the parental cells are nonsense transfected. Both cell lines are then exposed to the company's compound library. When stimulation is observed in the oGPCR transfected group and not in the nonsense group, a hit is obtained. This stimulation will produce a pattern, which can then be compared to the patterns present in the database, and thus the second messenger pathway utilized by the oGPCR is simultaneously determined.

Our method represents an extremely powerful tool for de-orphanizing GPCRs. With this method, we can supply information on hit identification and the pathway utilized by the oGPCR, simultaneously. No other single method or instrument on the market can accomplish this. This will provide tremendous savings in time and reagents for pharmaceutical companies interested in oGPCRs. All other systems presently on the market that cover this field, rely on molecular tags or transfected reporter gene systems and must do extensive work after hit identification to ascertain the second messenger pathway used by the oGPCR. None of this extra work is necessary with our method.

### CASE #3: DETERMINATION OF EC₅₀ VALUES AND RANK ORDER POTENCY FOR AGONISTS OF KNOWN AND UNKNOWN, TRANSFECTED AND ENDOGENOUS RECEPTORS

In this case we describe how our method and instrument can be used to determine EC₅₀ values and rank order potency for agonists.

For both known and unknown receptors, once a ligand has been identified and matched with its receptor, then determining EC₅₀ values is easily accomplished using our system. The ligand is added to the cells in increasing concentrations and the response is recorded over time. The kinetic information is recorded to determine when the response has reached a maximal value. The maximal impedance signal recorded at a set endpoint will be plotted against concentration added and the data fitted to sigmoidal curves. The EC₅₀ values are then calculated from the fitting equations. The rank order potency of a series of structurally similar agonists can be achieved by comparing the EC₅₀ values generated for each compound in separate experiments. Thus the same method and instrument that can identify the ligand and the receptor second messenger pathway can be used to generate EC₅₀ values and rank order potencies. In this case we are using only a small piece of the information that our method collects. However, the yield is highly useful pharmacological information.

The advantages of our method and instrument in this case are the same as listed for CASE #1. In addition, our method provides for the easy analysis of all kinds of endogenous receptors without the need for molecular tags, whereas all other methods work through the use of some form of label. This gives us the advantage of producing more physiologically relevant information. Still another advantage is the fact that we can accomplish this analysis in real-time without the need for cell lysis or fixation.
An additional advantage is that only one instrument is required to accomplish many tasks related to cellular response analysis.

### CASE #4: HIT IDENTIFICATION, DETERMINATION OF IC₅₀ VALUES, AND RANK ORDER OF POTENCY FOR ANTAGONISTS OF KNOWN AND UNKNOWN, TRANSFECTED AND ENDOGENOUS RECEPTORS

In this case we describe how our method and instrument can be used to do hit identification, determination of IC₅₀ values, and rank order potency for antagonists.

For both known and unknown receptors, once a ligand has been identified and matched with its receptor, determining hit identification and IC₅₀ values for antagonists is easily accomplished using our system. For antagonist hit identification, the company's compound library would be applied to cells containing the receptor of interest. After stimulation with a known or discovered ligand, hits would be revealed as attenuated responses. For IC₅₀ value determinations, antagonist is added to the cells at increasing concentrations and the response to a half maximal stimulating concentration of agonist is monitored over time. The kinetic information is recorded to determine when the inhibition has reached a maximal value. The minimal impedance signal, recorded at a set endpoint, will be plotted against the inhibitor concentration added, and the data fitted to sigmoidal curves. The IC₅₀ values are then calculated from the fitting equations. The rank order potency of a series of structurally similar antagonists can be achieved by comparing the IC₅₀ values generated for each compound in separate experiments. Thus the same method and instrument that can identify the ligand and the receptor second messenger pathway can be used to do antagonist hit identification, generate IC₅₀ values, and do rank ordering of inhibitors for potency. In this case again we are using only a small piece of the information that our method collects. However, the yield is highly useful pharmacological information.

Same as listed under Case #3.

### CASE #5: IDENTIFICATION OF EFFECTORS OF APOPTOSIS

In this case we describe how or method and instrument can be used for identification of effectors of apoptosis.

In this case we establish a database of apoptotic patterns (a training set) for HEK293 cells (or any other cell line, depending on the need of the customer) and include patterns produced during stimulation with known effectors of apoptosis. During the use of any unknown compound from a company's compound library, patterns generated would be compared to the apoptosis patterns generated in the training sets. If a match were made to one of these patterns, then a new effector of apoptosis would be discovered. This analysis could be done separately or in conjunction with agonist or antagonist fishing. This would be especially useful during antagonist fishing, since the information could be obtained simultaneously during data acquisition and any stimulators of apoptosis could be eliminated immediately as potential drug candidates. Thus tremendous amounts of time and effort could be saved in the drug discovery process.

Our method provides for the simultaneous analysis of apoptotic potential for any compound tested and therefore manifests a tremendous amount of savings for the drug discovery process as potential candidates that stimulate apoptosis can be eliminated early on in the process.

### CASE #6: IDENTIFICATION OF EFFECTORS OF CYTOTOXICITY

In this case we describe how or method and instrument can be used for identification of effectors of cytotoxicity.

In this case we establish a database of cytotoxic patterns (a training set) for HEK293 cells (or any other cell line, depending on the need of the customer) and include patterns produced during stimulation with known effectors of cytotoxicity. During the use of any unknown compound from a company's compound library, patterns generated would be compared to the cytotoxicity patterns generated in the training sets. If a match were made to one of these patterns, then a new effector of cytotoxicity would be discovered. This analysis could be done separately or in conjunction with agonist or antagonist fishing. This would be especially useful during antagonist fishing, since the information could be obtained simultaneously during data acquisition and any cytotoxic compounds could be eliminated immediately as potential drug candidates. Thus tremendous amounts of time and effort could be saved in the drug discovery process.

Our method provides for the simultaneous analysis of cytotoxic potential for any compound tested and therefore manifests a tremendous amount of savings for drug discovery as potential candidates that are cytotoxic can be eliminated early on in the process.

### CASE #7: ANALYSIS OF INTEGRATED CELLULAR RESPONSES TO MULTIPLE STIMULI

In this case we describe the use of our method for the analysis of integrated cellular responses to multiple stimuli.

The patterns that are generated with our method are representations of complex integrated cellular events that take place in and around the cell after stimulation. For this case we are trying to determine what will be the effect of one stimulus on another. For example, if we stimulate a known Gi GPCR receptor, and while the cell is responding to this agonist, we apply a ligand for a PTKR, what would the effect be? Since our method generates representations of integrated cellular events, we will be able to study these integrations with as many stimuli as we want. First, we establish sets of database patterns that represent stimulation of discreet pathways within a cell type, such as already discussed for GPCRs. Then, before, during, or after stimulation with a ligand or stimulator, we add a second ligand or stimulator and analyze the effect produced on the pattern elicited by the first ligand or stimulator. We are in the process of making correlations between individual Legendre parameters and unique cellular physiological events. Thus under the influence of multiple stimuli we will be able to ascertain which cellular physiological events are maintained and which altered when more than one stimuli is applied to a cell. This is extremely important in the drug discovery process as ultimately all drugs are to be used in complex individuals whose cells are inundated with multiple stimuli on a constant basis.

Our method allows for the analysis of integrated cellular responses without the use of pathway labels or any other manipulations. No other method can accomplish this in real-time without the use of labels.

### CASE #8: ANALYSIS OF TUMOR CELL PROGRESSION

In this case we describe how our method can be used to analyze tumor cell progression.

Tumor cells start out as relatively benign cells and as they divide and multiply, become more and more aggressive and ultimately become highly metastatic. When this occurs, the tumor cells break free of any restraints that they still have and spread throughout the body. This process is termed progression and ultimately leads to the death of the patient. As this process occurs the cells respond increasingly differently to the same stimuli as compared to earlier progeny. Again since our method produces an integrated representation of the cell, as the tumor cell progresses it should produce different patterns to the same stimuli. In this case, databases of patterns to certain ligands or stimuli will be created. As the tumor cells progress, the same ligands will be reapplied and the changes in the patterns recorded. Thus when test tumor cells are interrogated with our method, their pattern of response can be compared to the database and a determination of their state of progression can be made. Thus we have created an analysis tool to classify the state of progression of tumor cells.

Our method allows for the analysis of tumor cell progression without the use of labels or any other manipulations. No other method can accomplish this in real-time without the use of labels.

### CASE #9: DETERMINATION OF STEM CELL DIFFERENTIATION

In this case we describe how our method can be used to analyze stem cell differentiation.

Stem cells start out as pluri-potential cells with the ability to differentiate into any cell type. As the cell receives different stimuli from its environment, it changes the complement of expressed proteins it has and changes its ultimate function. At some point this process stops and the cell is locked into performing this one function. Thus it has differentiated to become a liver cell or a cartilage cell or a cardiac cell. Again since our method produces an integrated representation of the cell, as the stem cell differentiates, it should produce different patterns to the same stimuli. In this case, databases of patterns to certain ligands or stimuli will be created. As the stem cell differentiates, the same ligands will be reapplied and the changes in the patterns recorded. Thus, when test stem cells are interrogated with our method, their pattern of response can be compared to the database and a determination of their state of differentiation can be made. Thus we have created an analysis tool to classify what a particular stem cell will differentiate into.

Our method allows for the analysis of stem cell differentiation without the use of labels or any other manipulations. No other method can accomplish this in real-time without the use of labels.

### SUMMARY

In summary, we have demonstrated that this method can easily classify pathways stimulated by both transfected as well as endogenous receptors. The invention encompasses the process of pathway classification and incorporates the ability of the device and algorithms and software to classify unknown ligands as interacting with a number of cell surface receptors. This has been demonstrated through experimentation involving a Gi, Gs, or Gq GPCR or a PTKR. A unique aspect of the invention is the combination of processes that lead to classification of signal transduction pathways without the need for any kind of label or special amplification mechanism. The cell itself acts as the amplifying unit. In one embodiment, adherent eukaryotic cells were used with this system. An alternative embodiment incorporates the use of prokaryotes. Further alternative embodiments may incorporate either eurkaryotic or prokaryotic cell types as well as non adherent cells. In addition, the various embodiments of the method have the potential to classify any global cellular event as long as there are unique changes in the dielectric properties of cells after stimulation and therefore is not limited to the aforementioned pathways.

This technology has demonstrated applications in a variety of areas, including:
1. The detection of receptor-specific activation for many receptor & cell types -
   A. Surface, cytoplasmic, and nuclear receptors
   B. GPCRs (Gi, Gs- Gq-coupled and mixed GPCRs), protein tyrosine kinase receptors, and steroid receptors
   C. Endogenous & transfected receptors
   D. Adherent layer of cells
   E. Suspension cells sedimented to layer on an electrode
2. The generation of concentration response curves to generate EC₅₀/IC₅₀ values to rank compound potency
3. The identification of receptor-specific agonists and antagonists
4. The generation of response patterns which are representative or predictive of any specific receptor family or sub-family such as for the 3 GPCR families, the PTK receptor family, the cytokine receptor family (working through Smads, or Jak/STAT, or NF-kappa B), the nuclear receptor family, or the ion-channel receptor family.
5. The detection of blocking and/or modulation of subsections of biochemical signaling pathway involved in generating receptor-specific activation with chemical modulators that inhibit specific molecules or cellular events
6. The detection of non-receptor-based cellular activation
7. The deorphanization of receptors and ligands by:
   a. identifying ligands/agonists/antagonists of orphan receptor & determine receptor family (e.g. Gq-GCPR) & signaling pathway
8. The prediction of MOA (mechanism of action) for a receptor or of a compound
9. The detection and measurement of cell death & mode of death (e.g. apoptosis vs necrosis, specific pathways of apoptosis (e.g. bcl-2-dependent vs bcl-2-independent apoptosis))
10. The detection of integrated cellular response to multiple stimuli.
11. The detection of stem cell differentiation
12. The detection of tumor cell progression

While the above is a complete description of possible embodiments and applications of the inventive method, various alternatives, modifications and equivalents may be used. Further all publications and patent documents recited in this application are incorporated by reference in their entirety for all purposes to the same extent as if each individual publication and patent document was so individually denoted.

## Claims

1. A label-free method for the classification of cellular events, wherein the classification is achieved through measuring changes in the electrical properties of cells incorporated into an electrical circuit after application of a stimulus, the method comprising:
(a) measuring a value of an electrical property after application of a plurality of frequencies of the electromagnetic spectrum within a range of frequencies for selected time points during a selected period of time for the electrical circuit comprising cells with a receptor having a known receptor type and a known messenger pathway;
(b) selecting a reference time point corresponding to a time period immediately prior to addition of a known receptor stimulus;
(c) adding the known stimulus to the electric circuit such that the stimulus is able to interact with the receptor;
(d) calculating changes in the value of the electrical property for each frequency by subtracting the value of the measured electrical property for the reference time point from the value of the measured electrical property for each time point subsequent to the reference time point;
(e) parameterizing the changes in the value of the electrical property for each time point, receptor, and stimulus; and
(f) assigning the parameterized changes in value of the electrical property to a known stimulus/receptor interaction class.

2. The method of claim 1 further comprising the steps of
g) measuring the value of the electrical property for the electric circuit comprising cells for a plurality of frequencies in a range of frequencies for selected time points during a selected period of time for a cell with a known receptor type;
h) selecting a reference time point corresponding to a time period immediately prior to addition of an unknown stimulus ;
i) adding the unknown stimulus to the circuit such that the stimulus is able to interact with the receptor;
j) calculating changes in the value of the electrical property for each frequency by subtracting the value of the electrical property for the reference time point from the value of the electrical property for each time point subsequent to addition of the unknown stimulus;
k) parameterizing the changes in the value of the electrical property for each time point, receptor and stimulus;
l) comparing the parameterized changes in the value of the electrical property after addition of the unknown stimulus to the parameterized changes in value for known stimuli to correlate the changes in the value of the electrical property to a cellular response; and
m) assigning the cellular response to the unknown stimulus to a known substance/receptor interaction class and classifying the stimulus.

3. The method of claim 1 further comprising the steps of
g) measuring the value of the electrical property for the electrical circuit comprising cells for a plurality of frequencies within a range of frequencies for selected time points during a selected period of time for a cell with an unknown receptor type;
h) selecting a reference time point corresponding to a time period immediately prior to addition of a stimulus with a known response;
i) adding the stimulus to the circuit such that the stimulus is able to interact with the receptor;
j) calculating changes in the value of the electrical property for each frequency by subtracting the value of the electrical property for the reference time point from the value of the electrical property for each time point subsequent to addition of the stimulus;
k) parameterizing the changes in the value of the electrical property for each time point, receptor and stimulus;
l) comparing the parameterized changes in the value of the electrical property after addition of the stimulus to the parameterized changes in the value of the electrical properties for known receptors to correlate the changes in the value of the electrical property to a cellular response; and
m) assigning the cellular response of the unknown receptor to a known substance/receptor interaction class and classifying the receptor.

4. The method of claim 1, wherein the electrical property is complex impedance.

5. The method of claim 4 further comprising the steps of
g) measuring the complex impedance over a plurality of frequencies within a range of frequencies for each time point during a selected period of time for the electrical circuit comprising cells with a known receptor type;
h) selecting a reference time point corresponding to a time period immediately prior to addition of an unknown stimulus;
i) adding the unknown stimulus to the electrical circuit such that the stimulus is able to interact with the cell receptors;
j) calculating changes in the impedance for each frequency by subtracting the value of the impedance for the reference time point from the value of the impedance for each time point subsequent to addition of the unknown stimulus;
k) parameterizing the changes in complex impedance for each time point, receptor and stimulus;
l) comparing the parameterized changes in the complex impedance after addition of the unknown stimulus to the parameterized changes in the complex impedance for known stimuli to correlate the parameterized changes in the complex impedance to a cellular response; and
m) assigning the cellular response to a known substance/receptor interaction class and classifying the stimulus.

6. The method of claim 4 further comprising the steps of
g) measuring the complex impedance over a plurality of frequencies within a range of frequencies for each time point during a selected period of time for the electric circuit comprising cells with an unknown receptor type;
h) selecting a reference time point corresponding to a time period immediately prior to addition of a stimulus with a known response;
i) adding the stimulus to the electrical circuit such that the stimulus is able to interact with the cell receptors;
j) calculating changes in complex impedance for each frequency by subtracting the complex impedance for the reference time point from the complex impedance for each time point subsequent to addition of the stimulus;
k) parameterizing the changes in complex impedance for each time point, receptor and stimulus;
l) comparing the parameterized changes in complex impedance after addition of the stimulus to the parameterized changes in complex impedance for known receptors to correlate the parameterized changes in complex impedance to a cellular response; and
m)assigning the cellular response to a known substance/receptor interaction class and classifying the receptor.

7. The method of claim 4 wherein the changes in complex impedance are measured as resistance or reactance.

8. The method of claim 4 wherein the changes in complex impedance are measured as admittance, conductance, or susceptance.

9. The method of any one of claims 1 to 8, wherein the cellular events are chosen from the group comprising signal transduction from ligand/receptor interactions, cytotoxicity, apoptosis, tumor cell progression, and stem cell differentiation.

10. The method of any one of claims 1 to 9 wherein the electrical properties are chosen from the group comprising impedance phase, impedance magnitude, complex reflection coefficients, total circuit resistance, and total circuit capacitance.

11. The method of any one of the preceding claims, wherein the range of frequencies is 10Hz to 1000GHz.

12. The method of any one of the preceding claims, wherein the stimulus is a substance.

13. The method of claim 12, wherein the substance is a small molecule ligand.

14. The method of claim 12, wherein the substance is a ligand, a protein, an antibody, a lipid, a carbohydrate, a nucleic acid, water, or an ion.

15. The method of any one of the preceding claims, wherein the classification is achieved in real time.

16. The method of any one of the preceding claims, wherein the electrical circuit comprises interdigitated coplanar electrodes.

## Patentansprüche

1. Marker-freies Verfahren zum Klassifizieren zellulärer Ereignisse, wobei die Klassifizierung durch Messen von Änderungen der elektrischen Eigenschaften von Zellen, die in eine elektrische Schaltung einbezogen sind, nach dem Anwenden eines Reizes erreicht wird, wobei das Verfahren umfasst:
(a) Messen eines Werts einer elektrischen Eigenschaft nach dem Anwenden einer Mehrzahl von Frequenzen des elektromagnetischen Spektrums innerhalb eines Bereichs von Frequenzen für ausgewählte Zeitpunkte während eines ausgewählten Zeitraums für die elektrische Schaltung, die Zellen mit einem Rezeptor umfasst, die einen bekannten Rezeptortyp und einen bekannten Messenger-Weg aufweisen,
(b) Auswählen eines Bezugszeitpunkts, der einem Zeitraum unmittelbar vor dem Hinzufügen eines bekannten Rezeptorreizes entspricht,
(c) Hinzufügen des bekannten Reizes zu der elektrischen Schaltung derart, dass der Reiz mit dem Rezeptor eine Wechselwirkung eingehen kann,
(d) Berechnen von Änderungen des Werts der elektrischen Eigenschaft für jede Frequenz durch Subtrahieren des Werts der gemessenen elektrischen Eigenschaft für den Bezugszeitpunkt von dem Wert der gemessenen elektrischen Eigenschaft für jeden Zeitpunkt nach dem Bezugszeitpunkt,
(e) Parametrisieren der Änderungen des Werts der elektrischen Eigenschaft für jeden Zeitpunkt, Rezeptor und Reiz, und
(f) Zuordnen der parametrisierten Änderungen des Werts der elektrischen Eigenschaft zu einer bekannten Reiz/Rezeptor-Wechselwirkungsklasse.

2. Verfahren nach Anspruch 1, das ferner die Schritte
g) Messen des Werts der elektrischen Eigenschaft für die elektrische Schaltung, die Zellen umfasst, für eine Mehrzahl von Frequenzen in einem Bereich von Frequenzen für ausgewählte Zeitpunkte während eines ausgewählten Zeitraums für eine Zelle mit einem bekannten Rezeptortyp,
h) Auswählen eines Bezugszeitpunkts, der einem Zeitraum unmittelbar vor dem Hinzufügen eines unbekannten Reizes entspricht,
i) Hinzufügen des unbekannten Reizes zu der Schaltung derart, dass der Reiz mit dem Rezeptor eine Wechselwirkung eingehen kann,
j) Berechnen von Änderungen des Werts der elektrischen Eigenschaft für jede Frequenz durch Subtrahieren des Werts der elektrischen Eigenschaft für den Bezugszeitpunkt von dem Wert der elektrischen Eigenschaft für jeden Zeitpunkt nach dem Hinzufügen des unbekannten Reizes,
k) Parametrisieren der Änderungen des Werts der elektrischen Eigenschaft für jeden Zeitpunkt, Rezeptor und Reiz,
l) Vergleichen der parametrisierten Änderungen des Werts der elektrischen Eigenschaft nach dem Hinzufügen des unbekannten Reizes mit den parametrisierten Änderungen des Werts für bekannte Reize zum Korrelieren der Änderungen des Werts der elektrischen Eigenschaft mit einer zellulären Reaktion, und
m) Zuordnen der zellulären Reaktion auf den unbekannten Reiz zu einer bekannten Substanz/Rezeptor-Wechselwirkungsklasse und Klassifizieren des Reizes umfasst.

3. Verfahren nach Anspruch 1, das ferner die Schritte
g) Messen des Werts der elektrischen Eigenschaft für die elektrische Schaltung, die Zellen umfasst, für eine Mehrzahl von Frequenzen innerhalb eines Bereichs von Frequenzen für ausgewählte Zeitpunkte während eines ausgewählten Zeitraums für eine Zelle mit einem unbekannten Rezeptortyp,
h) Auswählen eines Bezugszeitpunkts, der einem Zeitraum unmittelbar vor dem Hinzufügen eines Reizes mit einer bekannten Reaktion entspricht,
i) Hinzufügen des Reizes zu der Schaltung derart, dass der Reiz mit dem Rezeptor eine Wechselwirkung eingehen kann,
j) Berechnen von Änderungen des Werts der elektrischen Eigenschaft für jede Frequenz durch Subtrahieren des Werts der elektrischen Eigenschaft für den Bezugszeitpunkt von dem Wert der elektrischen Eigenschaft für jeden Zeitpunkt nach dem Hinzufügen des Reizes,
k) Parametrisieren der Änderungen des Werts der elektrischen Eigenschaft für jeden Zeitpunkt, Rezeptor und Reiz,
l) Vergleichen der parametrisierten Änderungen des Werts der elektrischen Eigenschaft nach dem Hinzufügen des Reizes mit den parametrisierten Änderungen des Werts der elektrischen Eigenschaften für bekannte Rezeptoren zum Korrelieren der Änderungen des Werts der elektrischen Eigenschaft mit einer zellulären Reaktion, und
m) Zuordnen der zellulären Reaktion des unbekannten Rezeptors zu einer bekannten Substanz/Rezeptor-Wechselwirkungsklasse und Klassifizieren des Rezeptors umfasst.

4. Verfahren nach Anspruch 1, bei dem die elektrische Eigenschaft die komplexe Impedanz ist.

5. Verfahren nach Anspruch 4, das ferner die Schritte
g) Messen der komplexen Impedanz für eine Mehrzahl von Frequenzen innerhalb eines Bereichs von Frequenzen für jeden Zeitpunkt während eines ausgewählten Zeitraums für die elektrische Schaltung, die Zellen mit einem bekannten Rezeptortyp umfasst,
h) Auswählen eines Bezugszeitpunkts, der einem Zeitraum unmittelbar vor dem Hinzufügen eines unbekannten Reizes entspricht,
i) Hinzufügen des unbekannten Reizes zu der elektrischen Schaltung derart, dass der Reiz mit den Zellrezeptoren eine Wechselwirkung eingehen kann,
j) Berechnen von Änderungen der Impedanz für jede Frequenz durch Subtrahieren des Werts der Impedanz für den Bezugszeitpunkt von dem Wert der Impedanz für jeden Zeitpunkt nach dem Hinzufügen des unbekannten Reizes,
k) Parametrisieren der Änderungen der komplexen Impedanz für jeden Zeitpunkt, Rezeptor und Reiz,
l) Vergleichen der parametrisierten Änderungen der komplexen Impedanz nach dem Hinzufügen des unbekannten Reizes mit den parametrisierten Änderungen der komplexen Impedanz für bekannte Reize zum Korrelieren der parametrisierten Änderungen der komplexen Impedanz mit einer zellulären Reaktion, und
m) Zuordnen der zellulären Reaktion zu einer bekannten Substanz/Rezeptor-Wechselwirkungsklasse und Klassifizieren des Reizes umfasst.

6. Verfahren nach Anspruch 4, das ferner die Schritte
g) Messen der komplexen Impedanz für eine Mehrzahl von Frequenzen innerhalb eines Bereichs von Frequenzen für jeden Zeitpunkt während eines ausgewählten Zeitraums für die elektrische Schaltung, die Zellen mit einem unbekannten Rezeptortyp umfasst,
h) Auswählen eines Bezugszeitpunkts, der einem Zeitraum unmittelbar vor dem Hinzufügen eines Reizes mit einer bekannten Reaktion entspricht,
i) Hinzufügen des Reizes zu der elektrischen Schaltung derart, dass der Reiz mit den Zellrezeptoren eine Wechselwirkung eingehen kann,
j) Berechnen von Änderungen der komplexen Impedanz für jede Frequenz durch Subtrahieren der komplexen Impedanz für den Bezugszeitpunkt von der komplexen Impedanz für jeden Zeitpunkt nach dem Hinzufügen des Reizes,
k) Parametrisieren der Änderungen der komplexen Impedanz für jeden Zeitpunkt, Rezeptor und Reiz,
l) Vergleichen der parametrisierten Änderungen der komplexen Impedanz nach dem Hinzufügen des Reizes mit den parametrisierten Änderungen der komplexen Impedanz für bekannte Rezeptoren zum Korrelieren der parametrisierten Änderungen der komplexen Impedanz mit einer zellulären Reaktion, und
m) Zuordnen der zellulären Reaktion zu einer bekannten Substanz/Rezeptor-Wechselwirkungsklasse und Klassifizieren des Rezeptors umfasst.

7. Verfahren nach Anspruch 4, bei dem die Änderungen der komplexen Impedanz als Widerstand oder Reaktanz gemessen werden.

8. Verfahren nach Anspruch 4, bei dem die Änderungen der komplexen Impedanz als Scheinleitwert, Wirkleitwert oder Blindleitwert gemessen werden.

9. Verfahren nach einem der Ansprüche 1 bis 8, bei dem die zellulären Ereignisse aus der Gruppe, bestehend aus einer Signalübertragung von Ligand/Rezeptor-Wechseiwirkungen, Cytotoxizität, Apoptose, Tumorzellenprogression und Stammzellendifferenzierung, ausgewählt sind.

10. Verfahren nach einem der Ansprüche 1 bis 9, bei dem die elektrischen Eigenschaften aus der Gruppe, umfassend lmpedanzphase, lmpedanzgröße, komplexe Rückwirkungskoeffizienten, Gesamtwiderstand der Schaltung und Gesamtkapazität der Schaltung, ausgewählt sind.

11. Verfahren nach einem der vorstehenden Ansprüche, bei dem der Bereich der Frequenzen 10 Hz bis 1000 GHz beträgt.

12. Verfahren nach einem der vorstehenden Ansprüche, bei dem der Reiz eine Substanz ist.

13. Verfahren nach Anspruch 12, bei dem die Substanz ein kleiner Molekülligand ist.

14. Verfahren nach Anspruch 12, bei dem die Substanz ein Ligand, ein Protein, ein Antikörper, ein Lipid, ein Kohlenhydrat, eine Nukleinsäure, Wasser oder ein Ion ist.

15. Verfahren nach einem der vorstehenden Ansprüche, bei dem die Klassifizierung in Echtzeit erreicht wird,

16. Verfahren nach einem der vorstehenden Ansprüche, bei dem die elektrische Schaltung doppelkammförmige, koplanare Elektroden umfasst.

## Revendications

1. Procédé sans marquage pour la classification d'évènements cellulaires, dans lequel la classification est effectuée en mesurant des changements de propriétés électriques de cellules intégrées dans un circuit électrique après l'application d'un stimulus, le procédé comprenant :
(a) la mesure d'une valeur d'une propriété électrique après l'application d'une pluralité de fréquences du spectre électromagnétique dans une gamme de fréquences pour des instants sélectionnés pendant une période de temps choisie pour le circuit électrique comprenant des cellules avec un récepteur ayant un type de récepteur connu et une voie messagère connue ;
(b) la sélection d'un instant de référence correspondant à une période de temps située juste avant l'ajout d'un stimulus de récepteur connu ;
(c) l'ajout du stimulus connu au circuit électrique de telle sorte que le stimulus soit capable d'interagir avec le récepteur ;
(d) le calcul de changements de la valeur de la propriété électrique pour chaque fréquence en soustrayant la valeur de la propriété électrique mesurée pour l'instant de référence à la valeur de la propriété électrique mesurée pour chaque instant suivant l'instant de référence ;
(e) le paramétrage des changements de la valeur de la propriété électrique pour chaque instant, récepteur, et stimulus ; et
(f) l'attribution des changements paramétrés de la valeur de la propriété électrique à une classe d'interaction stimulus/récepteur connue.

2. Procédé selon la revendication 1, comprenant en outre les étapes consistant à
g) mesurer la valeur de la propriété électrique pour le circuit électrique comprenant des cellules pour une pluralité de fréquences dans une gamme de fréquences pour des instants sélectionnés pendant une période de temps choisie pour une cellule avec un type de récepteur connu ;
h) sélectionner un instant de référence correspondant à une période de temps située juste avant l'ajout d'un stimulus inconnu ;
i) ajouter le stimulus inconnu au circuit de telle sorte que le stimulus soit capable d'interagir avec le récepteur ;
j) calculer des changements de la valeur de la propriété électrique pour chaque fréquence en soustrayant la valeur de la propriété électrique pour l'instant de référence à la valeur de la propriété électrique pour chaque instant suivant l'ajout du stimulus inconnu ;
k) paramétrer les changements de la valeur de la propriété électrique pour chaque instant, récepteur et stimulus ;
l) comparer les changements paramétrés de la valeur de la propriété électrique après l'ajout du stimulus inconnu avec les changements paramétrés de valeur pour des stimuli connus afin de corréler les changements de la valeur de la propriété électrique à une réponse cellulaire ; et
m) attribuer la réponse cellulaire au stimulus inconnu à une classe d'interaction substance/récepteur connue et classifier le stimulus.

3. Procédé selon la revendication 1, comprenant en outre les étapes consistant à
g) mesurer la valeur de la propriété électrique pour le circuit électrique comprenant des cellules pour une pluralité de fréquences dans une gamme de fréquences pour des instants sélectionnés pendant une période de temps choisie pour une cellule avec un type de récepteur inconnu ;
h) sélectionner un instant de référence correspondant à une période de temps située juste avant l'ajout d'un stimulus avec une réponse connue ;
i) ajouter le stimulus au circuit de telle sorte que le stimulus soit capable d'interagir avec le récepteur ;
j) calculer des changements de la valeur de la propriété électrique pour chaque fréquence en soustrayant la valeur de la propriété électrique pour l'instant de référence à la valeur de la propriété électrique pour chaque instant suivant l'ajout du stimulus ;
k) paramétrer les changements de la valeur de la propriété électrique pour chaque instant, récepteur et stimulus ;
l) comparer les changements paramétrés de la valeur de la propriété électrique après l'ajout du stimulus aux changements paramétrés de la valeur des propriétés électriques pour des récepteurs connus afin de corréler les changements de la valeur de la propriété électrique à une réponse cellulaire ; et
m) attribuer la réponse cellulaire du récepteur inconnu à une classe d'interaction substance/récepteur connue et classifier le récepteur.

4. Procédé selon la revendication 1, dans lequel la propriété électrique est une impédance complexe.

5. Procédé selon la revendication 4, comprenant en outre les étapes consistant à
g) mesurer l'impédance complexe sur une pluralité de fréquences dans une gamme de fréquences pour chaque instant pendant une période de temps sélectionnée pour le circuit électrique comprenant des cellules avec un type de récepteur connu ;
h) sélectionner un instant de référence correspondant à une période de temps située juste avant l'ajout d'un stimulus inconnu ;
i) ajouter le stimulus inconnu au circuit électrique de telle sorte que le stimulus soit capable d'interagir avec les récepteurs de cellules ;
j) calculer les changements d'impédance pour chaque fréquence en soustrayant la valeur de l'impédance pour l'instant de référence de la valeur de l'impédance pour chaque instant suivant l'ajout du stimulus inconnu ;
k) paramétrer les changements d'impédance complexe pour chaque instant, récepteur et stimulus ;
l) comparer les changements paramétrés d'impédance complexe après l'ajout du stimulus inconnu avec les changements paramétrés d'impédance complexe pour des stimuli connus afin de corréler les changements paramétrés d'impédance complexe avec une réponse cellulaire ; et
m) attribuer la réponse cellulaire à une classe d'interaction substance/récepteur connue et classifier le stimulus.

6. Procédé selon la revendication 4, comprenant en outre les étapes consistant à
g) mesurer l'impédance complexe sur une pluralité de fréquences dans une gamme de fréquences pour chaque instant pendant une période de temps sélectionnée pour le circuit électrique comprenant des cellules avec un type de récepteur inconnu ;
h) sélectionner un instant de référence correspondant à une période de temps située juste avant l'ajout d'un stimulus avec une réponse connue ;
i) ajouter le stimulus au circuit électrique de telle sorte que le stimulus soit capable d'interagir avec les récepteurs de cellules ;
j) calculer les changements d'impédance complexe pour chaque fréquence en soustrayant l'impédance complexe pour l'instant de référence à l'impédance complexe pour chaque instant suivant l'ajout du stimulus ;
k) paramétrer les changements d'impédance complexe pour chaque instant, récepteur et stimulus ;
l) comparer les changements paramétrés d'impédance complexe après l'ajout du stimulus avec les changements paramétrés d'impédance complexe pour des récepteurs connus afin de corréler les changements paramétrés d'impédance complexe à une réponse cellulaire ; et
m) attribuer la réponse cellulaire à une classe d'interaction substance/récepteur connue et classifier le récepteur.

7. Procédé selon la revendication 4, dans lequel les changements d'impédance complexe sont mesurés comme une résistance ou une réactance.

8. Procédé selon la revendication 4, dans lequel les changements d'impédance complexe sont mesurés comme une admittance, une conductance ou une susceptance.

9. Procédé selon l'une des revendications 1 à 8, dans lequel les évènements cellulaires sont choisis parmi le groupe comprenant une transmission de signal issue d'interactions ligand/récepteur, une cytotoxicité, une apoptose, une évolution de cellule tumorale, et une différentiation de cellule souche.

10. Procédé selon l'une des revendications 1 à 9, dans lequel les propriétés électriques sont choisies parmi le groupe comprenant une phase d'impédance, une magnitude d'impédance, des coefficients de réflexion complexe, une résistance de circuit totale, et une capacité de circuit totale.

11. Procédé selon l'une des revendications précédentes, dans lequel la gamme de fréquences est de 10 Hz à 1000 GHz.

12. Procédé selon l'une des revendications précédentes, dans lequel le stimulus est une substance.

13. Procédé selon la revendication 12, dans lequel la substance est un petit ligand de molécule.

14. Procédé selon la revendication 12, dans lequel la substance est un ligand, une protéine, un anticorps, un lipide, un hydrate de carbone, un acide nucléique, de l'eau, ou un ion.

15. Procédé selon l'une des revendications précédentes, dans lequel la classification est effectuée en temps réel.

16. Procédé selon l'une des revendications précédentes, dans lequel le circuit électrique comprend des électrodes coplanaires interdigitées.
